(19)

Europäisches Patentamt
European Patent Office
Office européen des brevets

(11)   EP 4 492 305 A1

(12)   **EUROPEAN PATENT APPLICATION**
published in accordance with Art. 153(4) EPC

(43) Date of publication:
   **15.01.2025 Bulletin 2025/03**

(21) Application number: **23766832.2**

(22) Date of filing: **07.03.2023**

(51) International Patent Classification (IPC):
   **G06Q 10/06** (2023.01)

(52) Cooperative Patent Classification (CPC):
   **G06Q 10/06; G06Q 50/20**

(86) International application number:
   **PCT/JP2023/008517**

(87) International publication number:
   **WO 2023/171656 (14.09.2023 Gazette 2023/37)**

(84) Designated Contracting States:
   **AL AT BE BG CH CY CZ DE DK EE ES FI FR GB
   GR HR HU IE IS IT LI LT LU LV MC ME MK MT NL
   NO PL PT RO RS SE SI SK SM TR**
   Designated Extension States:
   **BA**
   Designated Validation States:
   **KH MA MD TN**

(30) Priority: **08.03.2022   JP 2022034992
               13.04.2022   JP 2022066524**

(71) Applicant: **Godot Inc.
   Kobe-shi, Hyogo 650-0035 (JP)**

(72) Inventors:
   • **MORIYAMA, Ken
     Kobe-shi, Hyogo 650-0035 (JP)**
   • **SUZUI, Go
     Kobe-shi, Hyogo 650-0035 (JP)**

(74) Representative: **Meissner Bolte Partnerschaft
   mbB
   Patentanwälte Rechtsanwälte
   Postfach 86 06 24
   81633 München (DE)**

(54)   **BEHAVIOR ASSISTANCE SYSTEM, BEHAVIOR ASSISTANCE METHOD, AND BEHAVIOR ASSISTANCE PROGRAM**

(57)   To provide a technique for more effectively supporting behavior change of a subject without using personal information of the subject or previously acquiring knowledge such as effective information provision for the subject. A behavior support system comprises: a storage unit that stores a plurality of respective message groups linked to a plurality of behavior change techniques that act on one or more behavior change factors of a plurality of behavior change factors, each message group including one or more messages; a generation unit that extracts a message from each of one or more message groups of the plurality of message groups and generates a message set including the extracted message for each subject; and a control unit that performs control so that the message in the message set is output to the subject in one or more batches.

Fig. 5

## Description

Cross-Reference to Related Applications

This application is based on Japanese Patent Application No. 2022-034992 filed on March 8, 2022 and Japanese Patent Application No. 2022-066524 filed on April 13, 2022 and claims the benefit of priority thereto, the contents of which are incorporated herein by reference in their entirety.

Technical Field

The present invention relates to a behavior support system, a behavior support method, and a behavior support program.

Background Art

In recent years, efforts to utilize approaches based on the theories of behavioral science that scientifically studies human behavior for service development have been spreading in various fields such as public policy, healthcare, retail, and education. There has also been considered a system that technically realizes supporting a subject's behavior change and habituation in the various fields (e.g., Patent Literature 1).

The system described in Patent Literature 1 describes: analyzing behavior data including various data measured for behaviors of a plurality of subjects, and based on a result of analysis of the behavior data, defining a stage indicator, which is an indicator as a criterion of a plurality of stages for aiming at a behavior as a target of habituation step by step, and each of the plurality of stages following the stage indicator; for each pair of adjacent stages, identifying a gap between two stages constituting the pair; for each stage pair, identifying a reason/measure that is at least one of a reason for existence of the identified gap and a measure for causing a subject belonging to a lower stage to make a behavior change to shift to a higher stage from relationship information in which a relationship between the gap and the reason/measure is defined; and executing processing related to the reason/measure identified for each stage pair.

Citation List

Patent Literature

Patent Literature 1

Japanese Patent Laid-Open No. 2020-140596

Summary of Invention

Technical Problem

In order to encourage behavior change of a subject, it may be effective to implement individualized interventions according to the behavioral characteristics of the subject using personal information such as the subject's attributes, income, or residential area. However, the following problems arise in order to use personal information of the subject. First, since the acquisition of personal information requires a high level of security and legal compliance, the hurdles to the implementation are high for both the provider and the recipient of the personal information. Second, the personal information required to actually identify the behavioral characteristics of the subject is often not clear in advance. Therefore, even if personal information is obtained by overcoming various hurdles, the personal information may not be available for the design and operation of a behavior change program. Third, the personal information obtained for intervention for behavior change is personal information of the subject at a point in time in the past, and does not necessarily show the state of the subject at the time of intervention. As a result, such intervention based on past personal information may not be able to produce the expected results.

Therefore, one of the objects of the present invention is to provide a behavior support system, a behavior support method, and a behavior support program that more effectively support behavior change of the subject without using personal information of the subject or previously acquiring knowledge such as effective approaches for the subject.

Solution to Problem

A behavior support system according to one aspect of the present invention is a behavior support system that supports behavior change of a subject, the behavior support system comprising: a storage unit that stores a plurality of respective message groups linked to a plurality of behavior change techniques that act on one or more behavior change factors of a plurality of behavior change factors, each message group including one or more messages; a generation unit that extracts a message from each of one or more message groups of the plurality of message groups and generates a message set including the extracted message for each subject; and a control unit that performs control so that the message in the message set is output to the subject in one or more batches.

According to this aspect, since a set of messages that act on a plurality of behavior change factors can be transmitted to the subject, it is possible to effectively encourage behavior change of the subject without using personal information of the subject or previously acquiring knowledge such as effective information provision for the subject.

In the above aspect, the generation unit may select a behavior change technique that acts only on a specific behavior change factor among the plurality of behavior change techniques for all of the plurality of behavior change factors, and extract one message from each of the respective message groups linked to the selected behavior change techniques to generate a first message set. According to this aspect, an exhaustive set of messages that act on the plurality of behavior change factors can be transmitted to the subject.

In the above aspect, the behavior support system further comprises: an acquisition unit that acquires response information to the output message from the subject; and an analysis unit that extracts a behavior change factor responded to by the subject from whom the response information has been acquired among the plurality of behavior change factors based on the response information, wherein the generation unit may further select, for the subject from whom the response information has been acquired, behavior change techniques that act on the behavior change factor responded to by the subject, and extract one or more messages from each of the respective message groups linked to the selected behavior change techniques to generate a second message set. According to this aspect, an intervention adapted to the influence of the passage of time or the change in environment on the behavior change factors can be performed for each subject, and it is possible to more effectively encourage behavior change of the subject.

In the above aspect, the generation unit may randomly extract a message from each message group.

In the above aspect, the plurality of behavior change factors may include capacity, opportunity, and motivation in a COM-B model.

In the above aspect, the plurality of behavior change factors may include at least two of an empirical attitude, an instrumental attitude, an injunctive norm, a descriptive norm, perceived control, self-efficacy, knowledge, a skill, salience of behavior, an environmental constraint, and habit.

A behavior support method according to another aspect of the present invention is a behavior support method for supporting behavior change of a subject, the behavior support method comprising: a step of extracting a message from each of one or more message groups of a plurality of respective message groups linked to a plurality of behavior change techniques that act on one or more behavior change factors of a plurality of behavior change factors, each message group including one or more messages, and generating a message set including the extracted message for each subject; and a step of performing control so that the message in the message set is output to the subject in one or more batches.

A behavior support program according to another aspect of the present invention is a behavior support program that a computer which supports behavior change of a subject is caused to execute, the behavior support program causing the computer to execute: a step of extracting a message from each of one or more message groups of a plurality of respective message groups linked to a plurality of behavior change techniques that act on one or more behavior change factors of a plurality of behavior change factors, each message group including one or more messages, and generating a message set including the extracted message for each subject; and a step of performing control so that the message in the message set is output to the subject in one or more batches.

Advantageous Effects of Invention

According to the present invention, it is possible to more effectively support behavior change of a subject without using personal information of the subject or previously acquiring knowledge such as effective information provision for the subject.

Brief Description of Drawings

[Figure 1] Figure 1 is a table illustrating a frame in which behavior change factors are associated with behavior change techniques in a matrix form.
[Figure 2] Figure 2 is a diagram conceptually illustrating a data structure of nudge message groups.
[Figure 3] Figure 3 is a diagram showing an example of a schematic configuration of a behavior support system according to the present embodiment.

[Figure 4] Figure 4 is a diagram showing an example of a hardware configuration of a device in the behavior support system according to the present embodiment.

[Figure 5] Figure 5 is a diagram showing an example of a functional configuration of the device in the behavior support system according to the present embodiment.

[Figure 6] Figure 6 is a flowchart showing an example of operation of the behavior support system according to the present embodiment.

[Figure 7] Figure 7 is a schematic diagram showing a specific example of MECE-type nudge cocktails.

[Figure 8] Figure 8 is a schematic diagram showing a specific example of a MECE-type nudge cocktail.

[Figure 9] Figure 9 is a schematic diagram showing a specific example of an adaptive nudge cocktail.

Description of Embodiments

Embodiments of the present invention will be described with reference to the accompanying drawings. Note that in each figure, components with the same reference numeral have the same or similar configuration.

(Outline of Behavior Support Method)

A behavior support method according to the present embodiment encourages behavior change by transmitting, to a subject for intervention, a nudge message that effectively acts on behavior change factors of the subject while scientifically and objectively searching for behavior change factors that are important for the subject. At this time, this embodiment uses a frame in which a plurality of behavior change factors are associated with a plurality of behavior change techniques that act on one or more behavior change factors of the plurality of behavior change factors.

<Frame>

Here, a behavior change factor is a factor that causes the subject to take a specific action. As behavior change factors, a plurality of behavior change factors in the target field can be identified based on surveys such as academic surveys or awareness surveys, theories related to behavioral science, persona setting of the subject, behavioral process maps, or the like.

Examples of behavior change factors can include "Capacity", "Opportunity", and "Motivation" in the COM-B model, which is commonly used in behavioral science. Alternatively, as other examples of behavior change factors, experiential attitudes, instrumental attitudes, injunctive norms, descriptive norms, perceived control, self-efficacy, knowledge, skills, salience of the behavior, environmental constraints, and habit are also known as the factors defined in the Integrated Behavioral Model (IBM) (also referred to as "IBM factors"), and in the present embodiment, behavior change factors including at least two of them may be identified.

Note that each of the above-described IBM factors is only an example, and higher-level factors that encompass the IBM factors may be specified as behavior change factors. For example, the above-mentioned experiential attitudes and instrumental attitudes may be encompassed by "Attitudes", the above-mentioned injunctive norms and descriptive norms may be encompassed by "Perceived norms", the above-mentioned perceived control and self-efficacy may be encompassed by "Personal Agency", the above-mentioned knowledge and skills may be encompassed by "Knowledge", salience of the behavior may be encompassed by "Importance", and environmental constraints may be encompassed by "Friction". Alternatively, lower-level factors into which the above IBM factors are divided may be specified as behavior change factors.

Of course, in the present embodiment, any factors related to behavioral intention such as factors defined by a behavioral model other than the COM-B model and IBM may be adopted as behavior change factors.

Means or techniques that act (work) on the behavior change factors as described above are called "Behavior Change Techniques (BCTs)". Alternatively, they may also be called intervention functions. Hereinafter, these means or techniques are also referred to as "BCTs".

BCTs (or intervention functions) that act on behavior change factors are theoretically known. For example, Lou Atkins and Susan Michie (UCL Centre for Behaviour Change, University College London), "Designing interventions to change eating behaviours" discloses a matrix showing the relationship between behavior change factors in the COM-B model and intervention actions (see "Table 2. Matrix of links between COM-B model and intervention functions").

The frame used in the present embodiment may be one obtained by applying the theory exemplified above, or may be developed based on surveys such as academic surveys or awareness surveys, persona setting of the subject, or behavioral process maps, in addition to the theory.

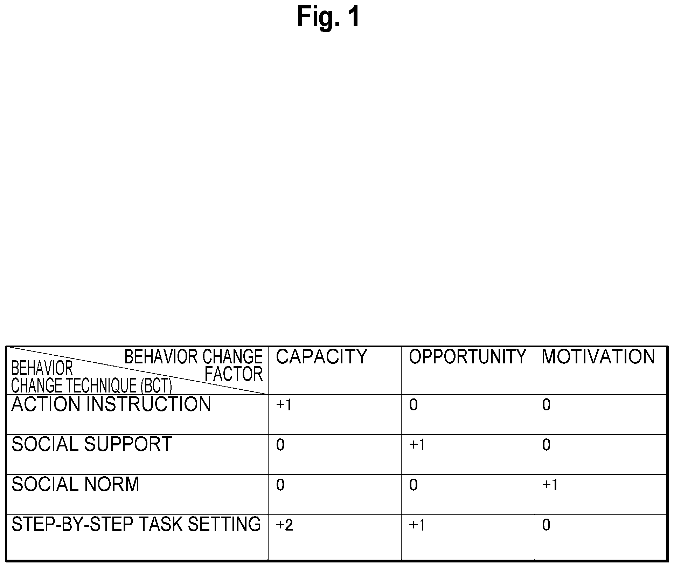

Figure 1 is a table illustrating a frame in which behavior change factors are associated with behavior change techniques in a matrix form. In Figure 1, the intensity of the action of five BCTs: "action instruction", "social support", "social norm", "step-by-step task setting", and "behavior observation" on three behavior change factors: "capacity", "opportunity", and "motivation" is expressed using the vector form. In the matrix shown in Figure 1, the sign "+" indicates that an action is observed, and the numerical value indicates the strength of the action.

In Figure 1, for example, it is shown that "action instruction" as a BCT acts only on the behavior change factor "capacity". It is shown that "step-by-step task setting" strongly acts on the behavior change factor "capacity" (+1) and also on "opportunity" (+1). It is shown that "behavioral observation" does not act on any of the three behavior change factors shown in Figure 1.

Hereinafter, a vector that expresses the effect of each BCT on the behavior change factors is also referred to as a "BCT vector". A specific BCT vector is also referred to as an "action instruction vector", a "social support vector", etc., using the name of the BCT. As exemplified in Figure 1, the action instruction vector for the three behavior change factors: "capacity", "opportunity", and "motivation" is expressed as <+1, 0, 0>.

<Nudge Message>

In the behavior support method according to the present embodiment, a plurality of BCTs that act on one or more behavior change factors of a plurality of behavior change factors related to the target field of intervention are identified, and a message group is linked to each of these BCTs. Each message group includes one or more nudge messages (hereinafter simply referred to as messages) that embody the linked BCT. For example, the BCT "action instruction" is linked to a message instructing an action, such as "Please do XX by the D-th day of the M-th month".

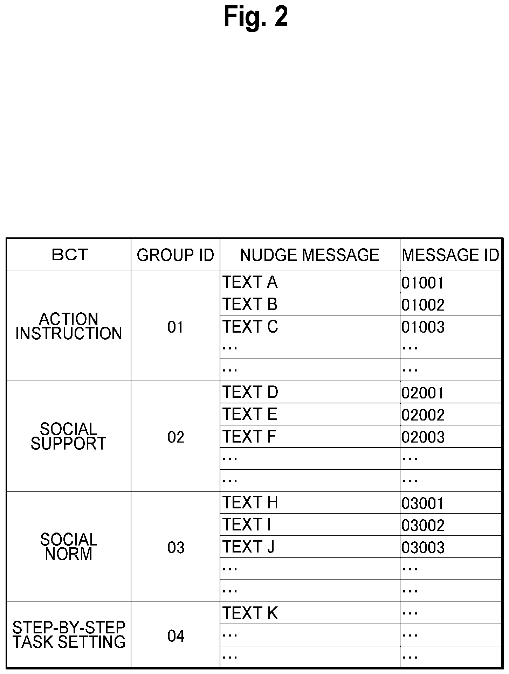

Figure 2 is a diagram conceptually illustrating a data structure of nudge message groups. In Figure 2, corresponding to the BCTs exemplified in Figure 1, a plurality of messages are linked to each of the five types of BCTs. Each message may be given an identification code (e.g., a message ID). The message group linked to each behavior change technique may be given a code (e.g., a group ID) for identifying the message group to which each message belongs.

When performing an intervention, one or more BCTs that act on behavior change factors identified according to the stage of the intervention among the plurality of behavior change factors related to the target field are selected, a message is extracted from each of the respective message groups linked to the selected BCTs, and a message set including the extracted messages is generated.

Extraction of a message from each message group is performed for each subject, preferably randomly. By incorporating randomness in the generation of a message set, selection bias of the intervention designer can be avoided, and stable intervention effects can be obtained compared to conventional intervention designs in which the intervention designer selects nudges based on their intuition and experience. This is because many intervention designers tend to use messages that they have used in the past or popular types of messages rather than scientific evidence.

However, it is conceivable that a message group includes a message that is considered to have an overwhelmingly greater impact on the subject than the other messages (the best message). In such a message group, random sampling of a message by equally treating the best message and the other messages may lead to a loss of opportunity for intervention. Therefore, a reinforcement learning method such as the Epsilon-Greedy method may be used to extract a message from each message group. Here, the Epsilon-Greedy method is a method of searching by acting randomly with a probability of $\varepsilon$ and selecting the action with the highest expected value with a probability of $(1 - \varepsilon)$. Specifically, the best message may be selected from a certain message group with a probability of $(1 - \varepsilon)$, and random extraction may be performed from the other messages with a probability of $\varepsilon$. Alternatively, one message may be randomly extracted from excellent messages that are equal to or greater than a certain threshold value with a probability of $(1 - \varepsilon)$, and the latest message may be extracted with a probability of $\varepsilon$.

Note that in some cases, only one message is linked to a certain BCT, in which case the one message is extracted from the BCT.

<Nudge Cocktail>

As described above, when performing an intervention, a message set is generated that consists of respective messages extracted from the message groups linked to one or more BCTs. Hereinafter, such a message set is referred to as a "nudge cocktail".

In the present embodiment, two types of nudge cocktails are generated depending on the stage of the intervention. The first nudge cocktail (first message set) is a nudge cocktail that is generated for all subjects in the initial stage of the intervention. Hereinafter, this first nudge cocktail is referred to as a MECE (Mutually Exclusive and Collectively Exhaustive)-type nudge cocktail.

The MECE-type nudge cocktail is composed of messages extracted from message groups linked to BCTs that act only on a specific behavior change factor among a plurality of BCTs that act on behavior change factors related to the target field. This is so that when a BCT has been accepted, it can be easily determined which behavior change factor has been acted on.

Such a MECE-type nudge cocktail is generated by selecting a BCT that acts only on a specific behavior change factor for all the behavior change factors related to the target field and extracting one message from each of the respective message groups linked to the selected BCTs. The reason to select a BCT that acts only on a specific behavior change factor for all the behavior change factors related to the target field is to cover the behavior change factors that can be responded to by the subject.

The second nudge cocktail (second message set) is a nudge cocktail generated based on behavior change factors adapted to the subject, and is generated for a subject who has shown some response to the MECE-type nudge cocktail. Hereinafter, this second nudge cocktail is referred to as an adaptive nudge cocktail.

The adaptive nudge cocktail is generated by identifying the behavior change factor responded to by the subject among the plurality of behavior change factors related to the target field based on the response from the subject, selecting behavior change techniques that act on the identified behavior change factor, and extracting one or more messages from each of the respective message groups linked to the selected behavior change techniques.

For both the MECE-type and adaptive nudge cocktails, the nudge cocktail is different for each subject because the nudge message is randomly extracted from each message group for each subject.

(Details of Behavior Support Method)

Next, a method of generating a nudge cocktail and a method of evaluating intervention in the behavior support method according to the present embodiment will be described.

<Coordinate System>

Here, in conventional behavior support methods, the process and reasons for the subject's behavior change due to the intervention have been qualitatively judged. However, in such methods, it has been difficult to design individualized interventions for each subject.

On the other hand, in the present embodiment, a coordinate system with a plurality of behavior change factors as axes is generated, and a target coordinate or a target area (hereinafter collectively referred to as a target area) is set in this coordinate system. Then, whether or not the subject has undergone behavior change is evaluated based on whether or not the coordinates of the subject have approached the target area in the coordinate system. Hereinafter, the distance calculated in this coordinate system is referred to as "Behavioral Scientific Distance (BSD)".

In such a coordinate system, by setting the current coordinates and the target area to be aimed at by interventions for each subject, the distance (BSD) from the current coordinates to the target area can be expressed as a multidimensional vector, and the BCT to be adopted to fill this distance can be objectively and clearly visualized.

Hereinafter, the frame shown in Figure 1 is used as an example to describe a procedure for intervention based on behavioral scientific distance.

(1) Selection of Coordinate Axes

First, based on academic surveys or awareness surveys, theories related to behavioral science, persona setting of the subject, or behavioral process maps, a plurality of behavior change factors in the target field are identified, and these factors are defined as coordinate axes. Hereinafter, among the behavior change factors exemplified in Figure 1, the coordinate axis indicating "capacity" is referred to as the C-axis, the coordinate axis indicating "opportunity" is referred to as the O-axis, and the coordinate axis indicating "motivation" is referred to as the M-axis.

Here, the units of the plurality of coordinate axes do not have to be common to each other, nor do they have to be integers. For example, when designing an intervention that encourages behavior change toward vaccination, each coordinate axis may be set such that the C-axis is a five-point scale, the O-axis is the number of times of access to the vaccination reservation site, and the M-axis is the number of seconds spent on the reservation site. The three-dimensional space defined by such a C-axis, O-axis, and M-axis is the coordinate system that expresses the process of behavior change of the subject.

(2) Setting of Target Area (Coordinates)

Next, the target area is set in the coordinate system consisting of the selected coordinate axes. The target area is set based on information, such as what kind of state the subject is in and what kind of intervention has caused the subject to take action, in the field of intervention. The target area may be set as pinpoint coordinates, or may be set as an expansive area such that "any value of the coordinates (c, o, m) is equal to or greater than 3". By setting the target area in this way, it is possible to clearly indicate the shortest distance to behavior change for each subject, according to the behavioral scientific characteristics in the field and the human characteristics of the subject.

The target area is set in common for all subjects. The influence of the passage of time, the change in environment or the like on behavior change factors can be easily expressed by changing the setting of the target area in the coordinate system. Note that influences on behavior change factors include, for example, changes in the level of a vaccination rate, improved convenience through improvements in the user interface (UI) or user experience (UX), and the approaching deadline for free medical examinations.

(3) Setting of Coordinates of Subject

For the above coordinate system, the initial coordinates of the subject are basically set to (c, o, m) = (0, 0, 0). However, for a subject for whom information such as a past reception history or attributes has been obtained, the initial coordinates may be set individually based on a certain coordinate conversion rule. For example, for a subject who has a past reception history, they understand the reception method (i.e., has the capacity), so the initial coordinates are set to (c, o, m) = (1, 0, 0).

In the present embodiment, an intervention action (such as transmission of a message) is determined or an intervention action is evaluated based on the coordinate system set in this way.

(4) Generation of MECE-Type Nudge Cocktail

A BCT that acts only on a certain behavior change factor is represented by a vector in which only one coordinate value has a value (non-zero) and the other coordinate values are zero. A MECE-type nudge cocktail is generated based on a collection of such BCT vectors.

Specifically, a collection is extracted such that the sum of the BCT vectors is <+1, +1, +1> and the number of constituent BCT vectors is the minimum value. Note that when a plurality of such collections are extracted, one of the collections is randomly selected for each subject. For example, in the case of the frame shown in Figure 1, a collection of three BCT vectors: an action instruction vector <+1, 0, 0>, a social support vector <0, +1, 0>, and a social norm vector <0, 0, +1> (a trivalent BCT vector) can be extracted. Further, for each subject, a MECE-type nudge cocktail consisting of three messages (a trivalent nudge cocktail) is generated by extracting one message from each of the message groups linked to the BCT vectors included in this collection. Note that the sum of the BCT vectors included in the collection may not be <+1, +1, +1>. For example, although the action instruction vector is <+1, 0, 0>, the social support vector is <0, +1, 0>, and the social norm vector is <0, 0, +1> in the frame of Figure 1, it is also possible to set the action instruction vector to <+1, 0, 0>, the social support vector to <0, +2, 0>, and the social norm vector to <0, 0, +3>, and extract the action instruction vector, the social support vector, and the social norm vector, whose sum is <+1, +2, +3>, as the collection. Thus, it is sufficient that the collection includes a predetermined number of BCT vectors and the sum of the predetermined number of BCT vectors is equal to a predetermined value. An n-valent nudge cocktail may be composed of messages corresponding to BCT vectors of n (n is an integer of 0 or more) types (i.e., behavior change factors).

(5) Update of Coordinates of Subject

When an intervention with a nudge cocktail is performed, the behavior change factor responded to by the subject is identified according to the response to the nudge cocktail, and the behavior change factor responded to by the subject for which the coordinate of the subject in the coordinate system is to be updated can be identified from the BCT accepted by the subject. For example, when the subject has accessed a reservation site guided by a message linked to a certain BCT, the BCT corresponding to the message is identified, and the behavior change factor acted on by this BCT can be identified using the frame exemplified in Figure 1.

For example, when a subject has accepted all BCTs for the MECE-type nudge cocktail described above, the state of the subject in the coordinate system is regarded to have changed from the initial coordinates (0, 0, 0) by the trivalent BCT vector (<+1, 0, 0>, <0, +1, 0>, <0, 0, +1>). That is, since the sum of changes due to the trivalent BCT vector is expressed as:

$$<+1, \ 0, \ 0> \ + \ <0, \ +1, \ 0> \ + \ <0, \ 0, \ +1> \ = \ <+1, \ +1, \ +1>,$$

these changes are added to the initial coordinates (0, 0, 0), so that the coordinates of the subject change to (1, 1, 1).

In this way, by updating the coordinates of the subject in the coordinate system, it is possible to objectively grasp the behavior change factor responded to by the subject and the distance to the target area.

(6) Generation of Adaptive Nudge Cocktail

An adaptive nudge cocktail is generated based on the current coordinates of each subject in the coordinate system. In detail, first, a vector representing the shortest route from the current coordinates of the subject to the nearest target area (hereinafter also referred to as an intervention vector) is calculated. When there are a plurality of candidates for the shortest route, a route may be randomly selected for each subject. Depending on the number of shortest routes, subjects for which interventions are continued may be selected by prioritizing them. For example, subjects who are away from the target area by a certain distance or more may be excluded from intervention.

Next, the intervention vector is decomposed to produce a collection of BCT vectors whose sum is equal to the intervention vector. For example, when the intervention vector is <+2, +2, +2>, candidates are the two types of collections:

$$[<+1, \ 0, \ 0> \times 2, \ <0, \ +1, \ 0> \times 2, \ <0, \ 0, \ +1> \times 2],$$

and

$$[<+2, \ +1, \ 0>, \ <0, \ +1, \ 0>, \ <0, \ 0, \ +1> \times 2].$$

If there are a plurality of candidates for a collection of BCT vectors equivalent to the intervention vector, a collection is randomly selected for each subject. If there is a constraint on intervention actions and the number of BCT vectors is limited (e.g., there is a constraint on the cost of transmitting messages and there is an upper limit on the number of messages that can be transmitted), a collection that satisfies the requirements is randomly selected from among the candidates. The set of respective messages extracted from the message groups linked to the BCT vectors constituting the selected collection becomes an adaptive nudge cocktail for the subject.

When an intervention with an adaptive nudge cocktail is performed, the behavior change factor responded to by the subject is identified according to the response to the nudge cocktail, and the coordinates of the subject in the coordinate system are updated, as described above. Then, if the coordinates of the subject have reached the target area, the intervention action is stopped. On the other hand, if the subject's coordinates have not yet reached the target area, an adaptive nudge cocktail is further generated based on the updated coordinates.

(Effects of Nudge Cocktails)

The reasons for generating two types of nudge cocktails: MECE-type and adaptive ones are as follows. That is, the behavior change factors of a subject are considered to be dynamic and change from day to day. Therefore, a nudge cocktail generated based on static personal information indicating the state of a subject at a certain point in time can make it difficult to encourage behavior change of the subject. In this regard, a MECE-type nudge cocktail is a combination of BCTs that act on a plurality of behavior change factors identified based on objective surveys or theories in a mutually exclusive and collectively exhaustive manner, and therefore it is possible to approach all behavior change factors related to the intervention from various perspectives.

On the other hand, an adaptive nudge cocktail consists of BCTs that act on the behavior change factor responded to by the subject who has received the MECE-type nudge cocktail. Therefore, by using an adaptive nudge cocktail, it is possible to take an approach to dynamically grasping the behavior change factors of the subject based on the response from the subject and intensively encouraging behavior change.

By combining such MECE-type and adaptive nudge cocktails, even if the behavior change factors of the subject are unknown at first, it is possible to grasp the behavior change factors of the subject in the process of sequentially performing interventions with these nudge cocktails and to effectively encourage behavior change.

Here, the number of messages constituting a nudge cocktail, in other words, the number of BCT vectors constituting a collection can be determined based on intervention design conditions such as the target level of the behavior change rate of the subject, the budget and time (period) that can be spent on interventions, ethical constraints, etc. Here, the ethical constraints are, for example, ethical considerations such as the fact that too many interventions lead to complaints from the subject or give the high-pressure impression to the subject. For example, if a trivalent nudge cocktail is expected to be effective for about 60% of all the subjects, while a pentavalent nudge cocktail is expected to be effective for about 90% of all the subjects, it is determined whether to use the trivalent or pentavalent one based on the intervention design conditions described above.

Note that in the generation of a nudge cocktail for the second time or later, messages are extracted from the remaining message groups excluding the messages transmitted to the subject in the past.

(Configuration of Behavior Support System)

Next, a configuration of the behavior support system according to the present embodiment will be described.

In the following, it is assumed that messages constituting a nudge cocktail are transmitted to the subject by e-mail or short message (SM) as a specific intervention action.

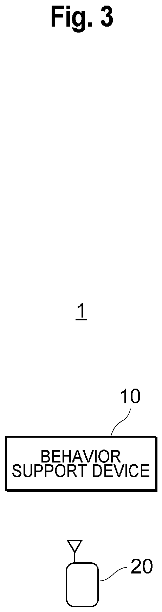

Figure 3 is a diagram illustrating an example of a schematic configuration of the behavior support system according to the present embodiment. As shown in Figure 3, the behavior support system 1 includes a behavior support device 10 and a terminal 20. The behavior support device 10 is an information processing device that provides the terminal 20 with a message using a nudge based on a behavior change factor that encourages behavior change of a subject.

The terminal 20 is a terminal used by a subject for behavior change, and it is sufficient that the terminal 20 is, for example, various information devices such as a smartphone, a mobile phone, a tablet, and a personal computer. The terminal 20 is something that can use a function for receiving messages such as short messages and e-mails, for example, various social network services (SNSs) such as LINE (registered trademark).

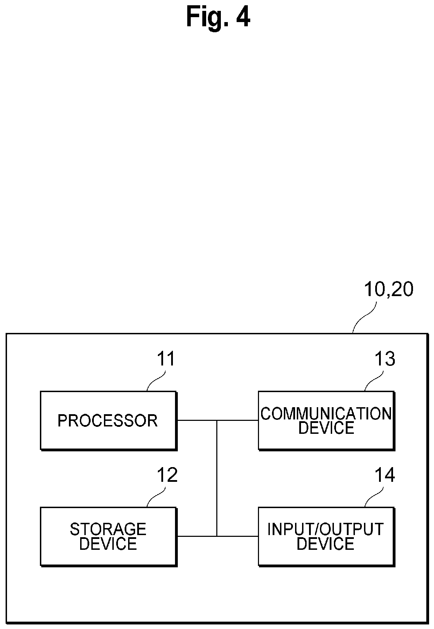

Figure 4 is a diagram illustrating an example of a hardware configuration of a device in the behavior support system 1. As shown in Figure 4, each device in the behavior support system 1 (e.g., each of the behavior support device 10 and the terminal 20) has a processor 11 such as a CPU (Central Processing Unit) corresponding to an arithmetic unit, a storage device 12, a communication device 13, and an input/output device 14. These components are connected to each other via a bus so as to be able to transmit and receive data.

The processor 11 is, for example, a CPU (Central Processing Unit), and is a control unit that performs control related to the execution of a program stored in the storage device 12, and the operation and processing of data. The processor 11 receives various input data from the input/output device 14 and/or the communication device 13, and outputs (e.g., displays) an operation result of the input data to the input/output device 14, stores it in the storage device 12, or transmits it via the communication device 13.

The storage device 12 is at least one of a memory, an HDD (Hard Disk Drive), and an SSD (Solid State Drive). The storage device 12 of the behavior support device 10 may store a behavior support program executed by the processor 11. The storage device 12 may be referred to as a "storage unit" or the like.

The communication device 13 is a device that performs communication via a wired and/or wireless network, and may include, for example, a network card, a communication module, a chip, and an antenna. The communication device 13 may be referred to as a "transmission unit" or a "receiving unit".

The input/output device 14 includes an input device such as a keyboard, a touch panel, a mouse and/or a microphone, and an output device such as a display and/or a speaker. The input/output device may be referred to as an "input unit" or an "output unit".

The hardware configuration described above is only an example. In each device in the behavior support system 1, a part of the hardware shown in Figure 4 may be omitted, or hardware not shown in Figure 4 may be provided. The hardware shown in Figure 4 may be composed of one or a plurality of chips.

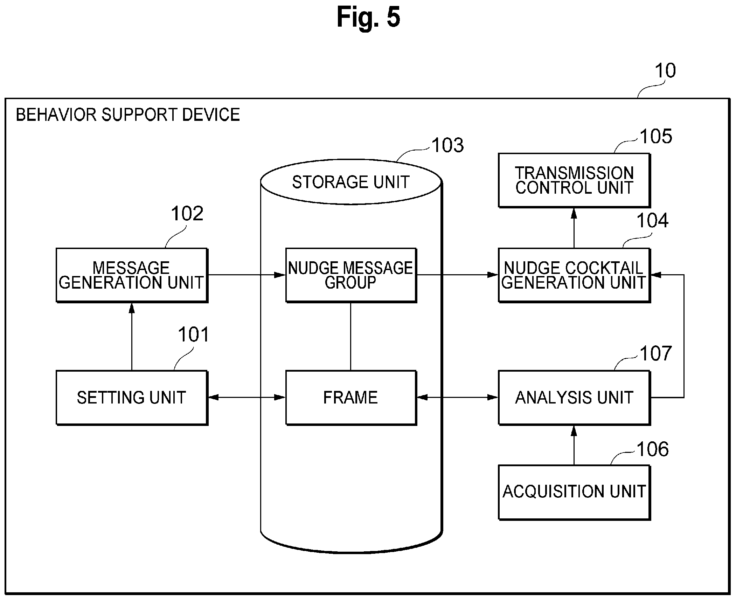

Figure 5 is a diagram showing a functional configuration of the device in the behavior support system 1. Note that Figure 5 is only an example, and each device in the behavior support system 1 may of course have functions not shown.

As shown in Figure 5, the behavior support device 10 includes a setting unit 101, a message generation unit 102, a storage unit 103, a nudge cocktail generation unit 104, a transmission control unit 105, an acquisition unit 106, and an analysis unit 107. Note that at least a part of the function implemented by the acquisition unit 106 can be implemented using the communication device 13. The setting unit 101, the message generation unit 102, the nudge cocktail generation unit 104, the transmission control unit 105, and the analysis unit 107 can be implemented by the processor 11 executing the behavior support program stored in the storage device 12. The behavior support program can be stored in a storage medium. The storage medium on which the program is stored may be a non-transitory computer readable medium. The non-transitory storage medium is not particularly limited, but may be, for example, a storage medium such as a USB memory or a CD-ROM. The storage unit 103 can be implemented using the storage device 12.

Note that in the present embodiment, the behavior support device 10 is shown as an integrated device, but may be divided into a plurality of devices. For example, the setting unit 101 and the message generation unit 102 may be configured as a separate device, and the separate device may be connected to the behavior support device 10 via a wired and/or wireless network.

The setting unit 101 sets a frame in which a plurality of behavior change factors are associated with a plurality of behavior change techniques that act on one or more behavior change factors of the plurality of behavior change factors, generates a coordinate system with the plurality of behavior change factors as axes, and makes various settings, such as the target area in the coordinate system and the initial setting of the coordinates of the subject.

The message generation unit 102 generates a plurality of respective message groups linked to a plurality of BCTs and stores them in the storage unit 103. Each message group includes one or more messages that specifically represent the linked BCT.

The message generation unit 102 may collect messages included in each message group via a wired or wireless communication network or a storage medium, or may automatically generate them by machine learning. Alternatively, automatically generated text edited by an operator may be stored as a message. The message generation unit 102 may store a message that is input as text from the input/output device 14, or may generate a message using a template prepared in advance.

The storage unit 103 stores a frame (see Figure 1) in which behavior change factors are associated with BCTs, a coordinate system generated by the setting unit 101, various setting information in the coordinate system, a nudge message group (see Figure 2) linked to each BCT, and the like.

The nudge cocktail generation unit 104 generates a nudge cocktail (a MECE-type nudge cocktail and an adaptive nudge cocktail) by extracting messages from nudge message groups stored in the storage unit 103 according to the stage of intervention.

The transmission control unit 105 performs control so that the messages included in the nudge cocktail generated by the nudge cocktail generation unit 104 are output to the subject in one or a plurality of batches. According to the control of the transmission control unit 105, the communication device 13 of the behavior support device 10 transmits a message to the terminal 20 of the subject. Messages can be transmitted by e-mail or SM (short message). Here, the message transmission control may include control of, for example, the order of transmitting a plurality of messages in a nudge cocktail, the date of transmitting each message, the time of day of transmitting each message, the time interval (transmission frequency) of transmitting the plurality of messages, the cancellation of transmission of each message, the number of times of transmitting each message, and the like.

The acquisition unit 106 acquires response information to an output message from the subject. The response information is information that represents a response from the subject who has received the message, and includes information such as whether a short message (SM) or email including a message has been read or not, a click through rate (CTR) for a link provided in an SM or email, and the time spent on the linked site. The acquisition unit 106 may acquire the response information from the terminal 20 of the subject in real time. The response information may also include information (e.g., a message ID) that identifies one or more messages responded to by the subject, thereby making it possible to determine the message group to which the message responded to by the subject belongs.

The analysis unit 107 analyzes the response information from each subject, and determines whether or not the intervention needs to be continued, the type of a nudge cocktail to be generated (MECE-type or adaptive), and the like according to the analysis result. Specifically, the analysis unit 107 extracts the behavior change factor responded to by the subject from whom the response information has been acquired among the plurality of behavior change factors.

(Operation of Behavior Support System)

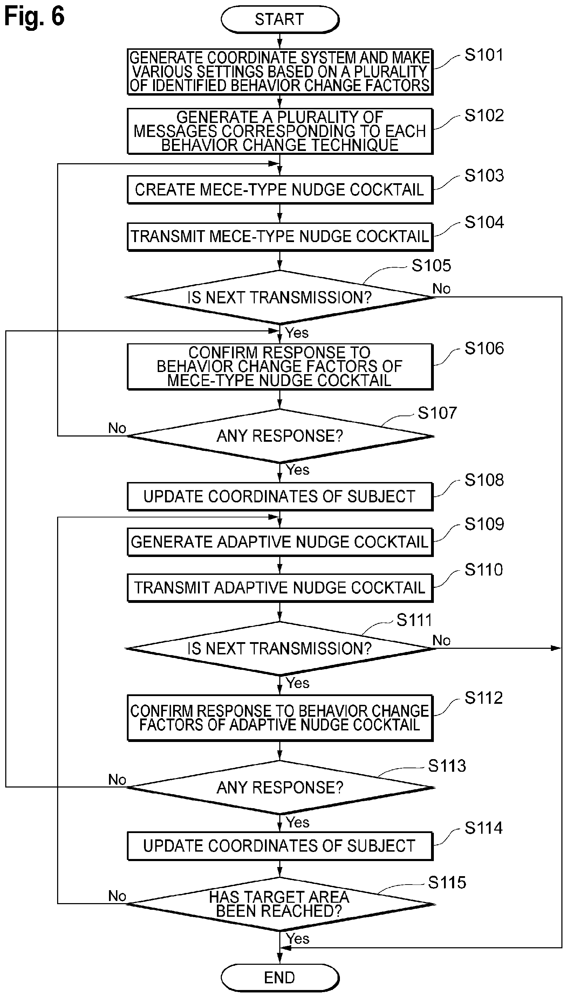

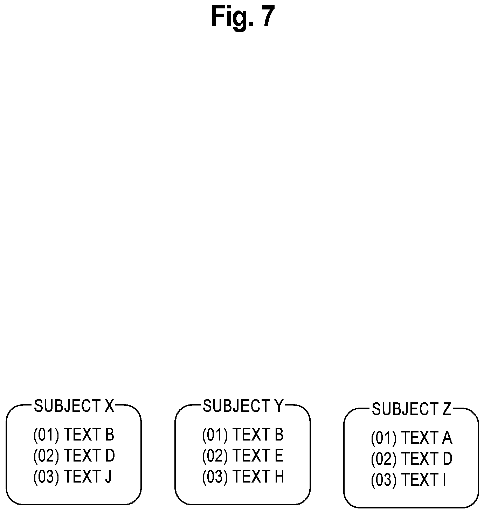

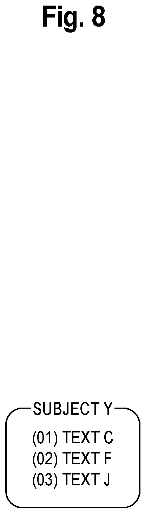

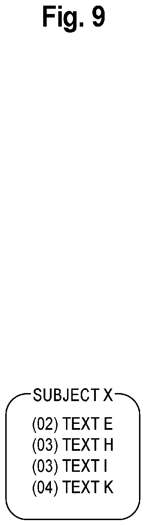

Figure 6 is a flowchart showing operation of the behavior support system according to the present embodiment. Note that the operation of the behavior support system shown in Figure 6 is only an example and is not limited to the one shown. Figures 7 and 8 are schematic diagrams illustrating a specific example of MECE-type nudge cocktails. Figure 9 is a schematic diagram illustrating a specific example of an adaptive nudge cocktail.

First, using various surveys or papers, a plurality of behavior change factors in the target field of intervention are identified in advance through the persona of a subject and behavioral process maps. Note that the identification of one or more behavior change factors is not limited to the above. For example, one or more behavior change factors may be identified by using a MECE-type nudge cocktail with a large valence. That is, it is also possible to narrow down the behavior change factors by the first intervention using a MECE-type nudge cocktail (determine the number of coordinate axes) and set the initial coordinates by the second intervention using a MECE-type nudge cocktail. The persona of the subject and/or behavioral process maps may be set based on data collected through payment applications or social network services (SNSs).

As shown in Figure 6, the behavior support device 10 generates a coordinate system and sets various settings based on the identified behavior change factors (step S101). That is, BCT vectors are defined based on a frame (see Figure 1) in which behavior change factors are associated with BCTs, a coordinate system with the plurality of behavior change factors as coordinate axes is generated, and the target area and the initial coordinates of the subject in the coordinate system are set.

Subsequently, the behavior support device 10 generates a plurality of messages corresponding to each BCT (behavior change technique) (step S102). The generated messages are stored in the storage unit 103 in association with the corresponding BCT.

Subsequently, the behavior support device 10 creates a MECE-type nudge cocktail for each subject (step S103). As described above, for the MECE-type nudge cocktail, a message is randomly extracted from each of the message groups linked to the plurality of BCTs, so that a different message set is created for each subject.

Specifically, as shown in Figure 7, for a subject X, a MECE-type nudge cocktail is generated that consists of the text B extracted from the message group linked to "action instruction", the text D extracted from the message group linked to "social support", and the text J extracted from the message group linked to "social norm" among the nudge message groups shown in Figure 2. Similarly, for a subject Y, a MECE-type nudge cocktail is generated that consists of the text B extracted from the message group linked to "action instruction", the text E extracted from the message group linked to "social support", and the text H extracted from the message group linked to "social norm". The same applies to a subject Z.

Subsequently, the behavior support device 10 transmits the generated MECE-type nudge cocktails to the respective subjects (step S104). At this time, all messages included in one nudge cocktail may be transmitted together, or one or more messages may be transmitted sequentially at certain time intervals.

Note that there may be a case where the number of BCTs constituting a nudge cocktail is smaller than the number of times of transmitting a message that is set in one intervention (an opportunity to generate a nudge cocktail). In such a case, the number of messages extracted from a message group linked to one type of BCT may be increased. For example, when the set number of times of transmission is 3 and the number of BCTs is 2, two messages may be randomly extracted from the message group linked to the BCT considered to have a relatively large impact. Alternatively, it is also possible to randomly extract one message from the message group linked to each BCT and further randomly extract one message from the entire two message groups (excluding the already-extracted message).

Instead of transmitting the messages included in a nudge cocktail by email or SM, the nudge cocktail may be transmitted in the form of a video or website that can be analyzed as a heatmap.

Thereafter, if there is no opportunity to further transmit a message (step S105: No), the behavior support device 10 ends the intervention action. On the other hand, when a message is further transmitted (step S105: Yes), the behavior support device 10 confirms the response to the behavior change factors of the transmitted MECE-type nudge cocktail for each subject (step S106).

The behavior support device 10 creates a MECE-type nudge cocktail again for a subject from whom any response to the MECE-type nudge cocktail has not been obtained (step S107: No). However, for a MECE-type nudge cocktail generated for the second time or later, one message is randomly extracted from each message group that is the extraction target except for the messages that have already been output to the subject. For example, if response information has not been acquired from the subject Y, then for the subject Y, as shown in Figure 8, a MECE-type nudge cocktail is generated that consists of one message (e.g., the text C) randomly extracted from the messages other than the already transmitted text B among the messages linked to "action instruction" (see Figure 2), one message (e.g., the text F) randomly extracted from the messages other than the already transmitted text E among the messages linked to "social support", and one message (e.g., the text J) randomly extracted from the messages other than the already transmitted text H among the messages linked to "social norm".

On the other hand, for a subject from whom some response has been obtained (step S107: Yes), the behavior support device 10 updates the coordinates in the coordinate system (step S108).

Subsequently, the behavior support device 10 generates an adaptive nudge cocktail for the subject (step S109). For example, if an intervention vector <+2, +2, +2> is calculated based on the updated coordinates of the subject X and a collection of BCT vectors [<+2, +1, 0>, <0, +1, 0>, <0, 0, +1> × 2] equivalent to this intervention vector is selected, then for the subject X, as shown in Figure 9, an adaptive nudge cocktail is generated that consists of one message (e.g., the text E) randomly extracted from the messages other than the already transmitted text D among the messages linked to "social support" (see Figure 2), two messages (e.g., the texts H and I) randomly extracted from the messages other than the already transmitted text J among the messages linked to "social norm", and one message (e.g., the text K) randomly extracted from the messages linked to "step-by-step task setting". Note that the valence n of the intervention vector may be equal to the number of types of BCT vectors included in the collection (i.e., the behavior change factors indicated by the BCT vectors). Thus, when the collection includes a plurality of BCT vectors of the same type (e.g., <0, 0, +1> as described above), messages as many as the plurality of BCT vectors may be extracted, or messages fewer than the plurality of BCT vectors may be extracted, from the viewpoint of diminishing effect and avoiding spam detection.

Subsequently, the behavior support device 10 transmits the generated adaptive nudge cocktail to each subject (step S110). The number of times of transmission and the transmission method are the same as in step S104.

Thereafter, if there is no opportunity to further transmit a message (step S111: No), the behavior support device 10 ends the intervention action. On the other hand, when further message transmission is performed (step S111: Yes), the behavior support device 10 confirms the response to the behavior change factors of the transmitted adaptive nudge cocktail for each subject (step S112).

For a subject from whom any response to the adaptive nudge cocktail has not been obtained (step S113: No), the behavior support device 10 confirms the response to the behavior change factors of the MECE-type nudge cocktail transmitted to the subject (step S106).

On the other hand, for a subject from whom some response to the adaptive nudge cocktail has been obtained (step S113: Yes), the behavior support device 10 updates the coordinates in the coordinate system (step S114). Then, if the updated coordinates have reached the target area (step S115: Yes), the behavior support device 10 stops intervention for the subject. On the other hand, if the updated coordinates have not reached the target area (step S115: No), the behavior support device 10 creates an adaptive nudge cocktail again for the subject (step S109).

As described above, according to the present embodiment, it is possible to effectively encourage behavior change of the subject without using personal information of the subject.

According to the present embodiment, since a MECE-type nudge cocktail enables non-overlapping exhaustive interventions from the beginning, it is possible to cause behavior change of the subject in a shorter time than before.

According to the present embodiment, even if the influence of the passage of time or the change in environment on the behavior change factors has occurred, such influence can be flexibly incorporated using an adaptive nudge cocktail.

According to the present embodiment, by incorporating randomness into the process of generating a nudge cocktail, stable intervention effects can be obtained compared to conventional intervention designs in which the intervention designer selects nudges based on their intuition and experience.

According to the present embodiment, quality can be ensured from aspects of behavioral science by identifying behavior change factors using the factors specified by the COM-B model or IBM. Further, since a message is randomly extracted from each message group to generate a nudge cocktail, it is possible to disperse the risk due to uncertainty that cannot be controlled by behavioral science.

Note that the results of behavior change achieved by applying the nudge cocktail generation method and the intervention evaluation method in the behavior support method according to the present embodiment have been demonstrated in various cases. For example, as shown in https://prtimes.jp/main/html/rd/p/000000091.000023382. htm l, Osaka City has identified the low rate of colorectal cancer screening (the rate in 2019 was 7.9%, and 6.7% in 2020) as an issue, and implemented a model project to encourage colorectal cancer screening for 4,000 citizens in a model implementation district in Osaka City based on the behavioral support method according to this embodiment, and as a result, the screening rate of the subjects reached 45.5%, far exceeding the target "screening rate of 20%".

The embodiments described above are intended to facilitate the understanding of the present invention and are not intended to be interpreted in a limited manner. The elements of the flowcharts, sequences, and embodiments described in the embodiment as well as their arrangements, materials, conditions, shapes, sizes, and the like are not limited to those exemplified, but can be modified as appropriate.

Reference Signs List

| | |
|---|---|
| 1 | behavior support system |
| 10 | behavior support device |
| 11 | processor |
| 12 | storage device |
| 13 | communication device |
| 14 | input/output device |
| 20 | terminal |
| 101 | setting unit |
| 102 | message generation unit |
| 103 | storage unit |
| 104 | nudge cocktail generation unit |
| 105 | transmission control unit |
| 106 | acquisition unit |
| 107 | analysis unit |

**Claims**

1. A behavior support system that supports behavior change of a subject, the behavior support system comprising:

   a storage unit that stores a plurality of respective message groups linked to a plurality of behavior change techniques that act on one or more behavior change factors of a plurality of behavior change factors, each message group including one or more messages;
   a generation unit that extracts a message from each of one or more message groups of the plurality of message groups and generates a message set including the extracted message for each subject; and
   a control unit that performs control so that the message in the message set is output to the subject in one or more batches.

2. The behavior support system according to claim 1,

   wherein the generation unit selects a behavior change technique that acts only on a specific behavior change factor among the plurality of behavior change techniques for all of the plurality of behavior change factors, and extracts one message from each of the respective message groups linked to the selected behavior change techniques to generate a first message set.

3. The behavior support system according to claim 1 or 2, further comprising:

an acquisition unit that acquires response information to the output message from the subject; and
an analysis unit that extracts a behavior change factor responded to by the subject from whom the response information has been acquired among the plurality of behavior change factors based on the response information, wherein the generation unit further selects, for the subject from whom the response information has been acquired, behavior change techniques that act on the behavior change factor responded to by the subject, and extracts one or more messages from each of the respective message groups linked to the selected behavior change techniques to generate a second message set.

4. The behavior support system according to any one of claims 1 to 3, wherein the generation unit randomly extracts a message from each message group.

5. The behavior support system according to any one of claims 1 to 4, wherein the plurality of behavior change factors include capacity, opportunity, and motivation in a COM-B model.

6. The behavior support system according to any one of claims 1 to 4, wherein the plurality of behavior change factors include at least two of an empirical attitude, an instrumental attitude, an injunctive norm, a descriptive norm, perceived control, self-efficacy, knowledge, a skill, salience of behavior, an environmental constraint, and habit.

7. A behavior support method executed in a behavior support device that supports behavior change of a subject, the behavior support method comprising:

a step of the behavior support device extracting a message from each of one or more message groups of a plurality of respective message groups linked to a plurality of behavior change techniques that act on one or more behavior change factors of a plurality of behavior change factors, each message group including one or more messages, and generating a message set including the extracted message for each subject; and
a step of the behavior support device performing control so that the message in the message set is output to the subject in one or more batches.

8. A behavior support program that a computer which supports behavior change of a subject is caused to execute, the behavior support program causing the computer to execute:

a step of extracting a message from each of one or more message groups of a plurality of respective message groups linked to a plurality of behavior change techniques that act on one or more behavior change factors of a plurality of behavior change factors, each message group including one or more messages, and generating a message set including the extracted message for each subject; and
a step of performing control so that the message in the message set is output to the subject in one or more batches.

# Fig. 1

| BEHAVIOR CHANGE FACTOR<br>BEHAVIOR CHANGE TECHNIQUE (BCT) | CAPACITY | OPPORTUNITY | MOTIVATION |
|---|---|---|---|
| ACTION INSTRUCTION | +1 | 0 | 0 |
| SOCIAL SUPPORT | 0 | +1 | 0 |
| SOCIAL NORM | 0 | 0 | +1 |
| STEP-BY-STEP TASK SETTING | +2 | +1 | 0 |

# Fig. 2

| BCT | GROUP ID | NUDGE MESSAGE | MESSAGE ID |
|---|---|---|---|
| ACTION INSTRUCTION | 01 | TEXT A | 01001 |
| | | TEXT B | 01002 |
| | | TEXT C | 01003 |
| | | … | … |
| | | … | … |
| SOCIAL SUPPORT | 02 | TEXT D | 02001 |
| | | TEXT E | 02002 |
| | | TEXT F | 02003 |
| | | … | … |
| | | … | … |
| SOCIAL NORM | 03 | TEXT H | 03001 |
| | | TEXT I | 03002 |
| | | TEXT J | 03003 |
| | | … | … |
| | | … | … |
| STEP-BY-STEP TASK SETTING | 04 | TEXT K | … |
| | | … | … |
| | | … | … |

# Fig. 3

1

10

BEHAVIOR
SUPPORT DEVICE

20

# Fig. 4

10,20

11 PROCESSOR

13 COMMUNICATION DEVICE

12 STORAGE DEVICE

14 INPUT/OUTPUT DEVICE

# Fig. 5

**BEHAVIOR SUPPORT DEVICE** (10)

- MESSAGE GENERATION UNIT (102)
- SETTING UNIT (101)
- STORAGE UNIT (103)
  - NUDGE MESSAGE GROUP
  - FRAME
- TRANSMISSION CONTROL UNIT (105)
- NUDGE COCKTAIL GENERATION UNIT (104)
- ANALYSIS UNIT (107)
- ACQUISITION UNIT (106)

EP 4 492 305 A1

**Fig. 6**

```
                    START

GENERATE COORDINATE SYSTEM AND MAKE      S101
VARIOUS SETTINGS BASED ON A PLURALITY
OF IDENTIFIED BEHAVIOR CHANGE FACTORS

GENERATE A PLURALITY OF                  S102
MESSAGES CORRESPONDING TO EACH
BEHAVIOR CHANGE TECHNIQUE

CREATE MECE-TYPE NUDGE COCKTAIL          S103

TRANSMIT MECE-TYPE NUDGE COCKTAIL        S104

                                         S105
IS NEXT TRANSMISSION?              No
            Yes

CONFIRM RESPONSE TO                      S106
BEHAVIOR CHANGE FACTORS OF
MECE-TYPE NUDGE COCKTAIL

                                         S107
No        ANY RESPONSE?
            Yes

UPDATE COORDINATES OF SUBJECT            S108

GENERATE ADAPTIVE NUDGE COCKTAIL         S109

TRANSMIT ADAPTIVE NUDGE COCKTAIL         S110

                                         S111
IS NEXT TRANSMISSION?              No
            Yes

CONFIRM RESPONSE TO BEHAVIOR CHANGE      S112
FACTORS OF ADAPTIVE NUDGE COCKTAIL

                                         S113
No        ANY RESPONSE?
            Yes

UPDATE COORDINATES OF SUBJECT            S114

                                         S115
No     HAS TARGET AREA
       BEEN REACHED?
            Yes

                    END
```

# Fig. 7

```
┌─SUBJECT X─┐      ┌─SUBJECT Y─┐      ┌─SUBJECT Z─┐
│                  │      │                  │      │                  │
│ (01) TEXT B      │      │ (01) TEXT B      │      │ (01) TEXT A      │
│ (02) TEXT D      │      │ (02) TEXT E      │      │ (02) TEXT D      │
│ (03) TEXT J      │      │ (03) TEXT H      │      │ (03) TEXT I      │
│                  │      │                  │      │                  │
└──────────────────┘      └──────────────────┘      └──────────────────┘
```

# Fig. 8

SUBJECT Y

(01) TEXT C
(02) TEXT F
(03) TEXT J

# Fig. 9

```
┌─SUBJECT X─┐
│ (02) TEXT E │
│ (03) TEXT H │
│ (03) TEXT I │
│ (04) TEXT K │
└───────────┘
```

## INTERNATIONAL SEARCH REPORT

| | International application No. |
|---|---|
| | **PCT/JP2023/008517** |

### A. CLASSIFICATION OF SUBJECT MATTER

*G06Q 10/06*(2023.01)i
FI:  G06Q10/06

According to International Patent Classification (IPC) or to both national classification and IPC

### B. FIELDS SEARCHED

Minimum documentation searched (classification system followed by classification symbols)

G06Q10/00-99/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 6935118 B1 (K THREE INC.) 15 September 2021 (2021-09-15) paragraphs [0063]-[0072] | 1-8 |
| A | JP 2021-086282 A (NIPPON TELEGRAPH AND TELEPHONE CORP.) 03 June 2021 (2021-06-03) abstract | 1-8 |
| A | JP 2020-140360 A (OKI ELECTRIC INDUSTRY CO., LTD.) 03 September 2020 (2020-09-03) abstract | 1-8 |

☐ Further documents are listed in the continuation of Box C.      ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **19 April 2023** | **09 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2023/008517**

| Patent document cited in search report | | | Publication date (day/month/year) | Patent family member(s) | | | Publication date (day/month/year) |
|---|---|---|---|---|---|---|---|
| JP | 6935118 | B1 | 15 September 2021 | JP | 2022-163957 | A | |
| JP | 2021-086282 | A | 03 June 2021 | (Family: none) | | | |
| JP | 2020-140360 | A | 03 September 2020 | (Family: none) | | | |

Form PCT/ISA/210 (patent family annex) (January 2015)

**EP 4 492 305 A1**

**Patent documents cited in the description**

- JP 2022034992 A
- JP 2022066524 A
- JP 2020140596 A